# EUROPEAN PATENT SPECIFICATION

(11) **EP 1 621 181 B1**
(45) Date of publication and mention of the grant of the patent: **08.09.2010**
(21) Application number: 05254275.0
(22) Date of filing: 07.07.2005
(51) Int. Cl.: A61K 8/25, A61K 8/39, A61K 8/86, A61K 8/92, A61K 8/06, A61Q 15/00

(54) **Antiperspirant or deodorant emulsion comprising a nonionic emulsifier and fumed silica**
Emulsionsförmige Antitranspirant- oder Deodorantzubereitung enthaltend einen nichtionischen Emulgator und pyrogenes Siliciumdioxid
Emulsion antitranspirante ou désodorante comprenant un agent émulsifiant nonionique et une silice pyrogénique

(30) Priority: 20.07.2004 GB 0416252
(43) Date of publication of application: 01.02.2006
(73) Proprietor: Unilever PLC, London EC4Y 0DY (GB); Unilever N.V., 3013 AL Rotterdam (NL)
(72) Inventor: Batista, Andrea Paula, Seacroft, Yorkshire, LS14 2AR (GB); Inger, Michael John, Bebington, Merseyside, CH63 3JW (GB); Pesa, Adrian Simon, Descartes, Tortuguitas, 4020 (AR); Volker, Axel, Seacroft, Yorkshire, LS14 2AR (GB)
(74) Representative: Whaley, Christopher

(56) References cited:
- WO-A-01/85117
- WO-A-03/015722
- WO-A-2004/064792
- DE-A1- 10 140 586
- DE-A1- 19 514 269
- US-A- 4 777 035
- US-A- 5 468 473
- HASENZAHL S ET AL: "FUMED SILICA FOR PERSONAL CARE AND COSMETICS - VERSATILE AND EFFECTIVE" SOFW-JOURNAL SEIFEN, OELE, FETTE, WACHSE, VERLAG FUR CHEMISCHE INDUSTRIE, AUGSBURG, DE, vol. 129, no. 8, August 2003 (2003-08), pages 1-8, XP002340152 ISSN: 0942-7694
- HARDY J F ET AL: "THE USE OF FUMED SILICA IN COSMETICS" COSMETIC TECHNOLOGY, CEC, MILAN,, IT, March 1980 (1980-03), pages 35-39, XP008019427 ISSN: 1127-6312

## Description

The present invention relates to a cosmetic composition, and in particular to antiperspirant or deodorant formulations suitable for controlling body odour or perspiration from localised areas of the body, such as in the underarm.

Sweating is a natural bodily function of humans which results in the formation of visible wet patches on human skin or in clothing which comes into contact with wet or damp skin. The appearance of such wet patches is considered undesirable in a number of societies in many circumstances and accordingly an industry has become established to produce materials, commonly called antiperspirants, which control sweating, especially in those skin regions where the density of sweat glands is particularly high, such as in the underarm.

Moreover, even though freshly secreted sweat tends to have little odour, the resident skin population of microorganisms and particularly certain stains of bacteria act on the secretions to transform at least a fraction of them into malodorous compounds, and especially in occluded sites in the body such as in the underarm. Accordingly, a parallel industry has become established in which compositions, commonly called deodorants, are contacted with the skin so as to mask such malodours or to prevent or retard their formation. By virtue of the suppression of sweating, antiperspirants usually additionally provide localised deodorancy.

Antiperspirant or deodorant dispensers commonly fall into one or other of two categories, namely contact or noncontact dispensers. Choice between those two categories tends to be a matter of personal preference since safe and effective formulations are available in either category. Within the category of contact dispensers, there is a range of alternative classes of dispenser depending on the physical characteristics of the composition, including sticks in which the composition forms a solid mass which can retain its integrity unsupported by a container, and soft solids or creams which are either very viscous or thixotropic which can be retained within a dispensing container but dispensed therefrom through apertures or slits by mild pressure. A third class of dispensers and compositions comprises liquids with a relatively low viscosity that commonly are dispensed via a roller that sits loosely enough within a housing forming an outlet from the dispenser to allow it to rotate when rolled across the skin, often called roll-ons.

The instant invention relates to compositions that can be dispensed by roll-ons. Many roll-on deodorant or antiperspirant compositions have been based on a low molecular weight alcohol such as ethanol as the principal carrier and/or active ingredient in the composition, by which herein we mean the ingredient providing the largest single weight fraction. It has bactericidal properties and is a good solvent to common antiperspirant actives. However, it has a number of characteristics which are disliked by or repugnant to a significant fraction of prospective deodorant or antiperspirant users. These characteristics include stinging, especially if the skin is broken or abraded, and a significant cooling effect. For that reason, since the perception by consumers of cosmetic compositions can influence to a considerable extent whether or not they purchase the same brand again, it is desirable to devise compositions for consumers who wish to avoid ethanol-based compositions. Some alternative formulations are aqueous emulsions but they can suffer from one or more unfavourable characteristics such as at least a perception of drying too slowly and/or greasiness.

WO03/041674 discloses a roll-on formulation of the water-in-oil type. Water-in oil formulations are known to the skilled man to present different problems from oil in water cosmetic formulations; for example, they act differently because the external phase is different. Not surprisingly, therefore, it is silent about the problems of drying indicated above.

US 2003/0108580 is directed to a process for the preparation of granulates of pyrogenically-produced silicon dioxide and to the use of such granulates in cosmetic compositions, exemplifying creams and powders. It is silent about the problems of drying oil-in-water emulsions.

US5849276 discloses the use of silica as a nucleating agent in anhydrous antiperspirant compositions. Silica has previously been incorporated into anhydrous antiperspirant stick formulations (JP55004355) and anhydrous deodorant compositions (JP01143820). Each of these three specifications is silent about the problems of drying emulsions indicated above.

In DEOS 1101 40 586 and WO 03/015722, there are disclosed oil in water emulsions containing an antiperspirant and a mixture of emulsifiers and a stabiliser in weight ratios being rational numbers from 1 to 3 and having a viscosity of from 100 to 1500 mPa.s..

In, DEOS 195 14 269, there are disclosed oil in water emulsions with a droplet size of 100 to 300 nm, comprising an astringent antiperspirant salt, an oil component and a combination of a non-ionic emulsifier having an HLB value of 10-15 and a co-emulsifier being a C₁₆₋₂₂ saturated fatty alcohol or a partial ester of C₃₋₆ polyol with a saturated fatty acid of C₁₄₋₂₂.

In WO 2004/064792, published on 5th August 2004, there are disclosed two phase antiperspirant or deodorant compositions comprising a non-polar phase comprising a silicone, 0-25% of a hydrocarbon polymer, 0-15% of low viscosity lipophilic emollientsd selected from hydrocarbons, benzoic acid esters, and propoxylated fatty alcohols, and optionally one or more ingredients selected from silicone surfactants, hydrophobically treated colloidal fumed silica, fragrance, vitamins and coloring agent and a polar phase comprising the antiperspirant active, an aqueous alcoholic component, a thickening agent and one or more optional ingredients selected from mica, a suspending agent, a masking agent, an ethylene oxide polymer,and an emulsifier with HLB ≥ 10, in which the ratio of oil phase to water phase is in a specified range and the compositioncan form a temporarily stabilized emulsion after shaking for a period not exceeding 24 hours.

In US 5486473, there are disclosed aqueous alcoholic antiperspirant compositions which in include a thickener, claiming the use in combination with hydroxyalkyl cellulose of 3.5 of 4.5% colloidal silica.

In a paper by S. Hasenzahl et al in the Internationales Journal für angewandte Wissenschaft (Sonderdruck aus 8-2003) entitled Fumed silica for personal care and cosmetics: versatile and effective, there are disclosedwater in oil compositions containing hydrophobic fumed silica on page 5 and antiperspirant composition containing hydrophobic fumed silica on page 8 that do not specify the nature of carrier materials for the antiperspirant.

In a paper by John F Hardy et al in Cosmetic Technology, March 1980, pp35-39, entitled "The use of fumed silica in cosmetics", the use of fumed silica as a thickener is disclosed.

In US 4777035, there are disclosed antiperspirant compositions in which a relatively large amount of water is encapsulated onto a specific kind of hydrophobic silica.

It is an objective to obtain a composition that is suitable for employment in a roll-on dispenser and which at least ameliorates one or more unfavourable or disliked characteristics of such compositions, such as one or more mentioned hereinbefore.

According to the present invention there is provided a cosmetic composition in accordance with claim 1 herein.

By the incorporation of hydrophobic particulate silica into such an emulsion, it is possible to improve or ameliorate one or more unfavourable characteristics of corresponding silica-free emulsions.

The instant invention employs an effective concentration of an antiperspirant or deodorant active, which is say that is sufficient to reduce or control sweating or reduce or eliminate body malodour. In many desirable embodiments, the composition contains at least 1% antiperspirant active, and preferably a least 5% and often is at least 10%. Commonly, the concentration of the antiperspirant active is not higher than 30%, and in many practical embodiments is not higher than 25.5%, %s herein being by weight based on the composition unless otherwise stated. A preferred concentration range for the antiperspirant active is from 10 to 20%.

The antiperspirant active is conveniently an astringent aluminium and/or zirconium salt, including astringent inorganic salts, astringent salts with organic anions and complexes of such salts. Preferred astringent salts include aluminium, zirconium and aluminium/zirconium halides and halohydrate salts, such as especially chlorohydrates. Activated chlorohydrates can be incorporated, if desired. Some literature employs alternative terminology for chlorohydrates, such as basic aluminium chloride, and aluminium chlorhydrex.

Aluminium halohydrates are usually defined by the general formula Al₂(OH)ₓQ_{y}·wH₂0 in which Q represents respectively chlorine, bromine or iodine, (and especially chlorine to form a chlorohydrate) x is variable from 2 to 5 and x + y = 6 while wH₂O represents a variable amount of hydration.

Zirconium actives can usually be represented by the empirical general formula: ZrO(OH)_{2n-nz}B_{z}.wH₂0 in which z is a variable in the range of from 0.9 to 2.0 so that the value 2n-nz is zero or positive, n is the valency of B, and B is selected from the group consisting of chlorine (to form a chlorohydrate), other halide, sulphamate, sulphate and mixtures thereof. Possible hydration to a variable extent is represented by wH₂0. Preferably, B represents chlorine and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes.

In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH.

In some compositions, it is highly desirable to employ complexes of a combination of aluminium chlorohydrates and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis.

The invention compositions can comprise, if desired, a deodorant active other than an antiperspirant active described hereinbefore. Such an alternative deodorant active can be selected conveniently from any deodorant active known in the cosmetic art such as antimicrobial actives such as polyhexamethylene biguanides, e.g. those available under the trade name Cosmocil™ or chlorinated aromatics, eg triclosan available under the trade name Irgasan™, non-microbiocidal deodorant actives such as triethylcitrate, bactericides and bacteriostatis. Yet other deodorant actives can include bactericidal zinc salts such as zinc ricinoleate. The concentration of such alternative deodorant active is desirably from 0.01 to 5% and in many instances is from 0.1 to 1% by weight of the composition.

In many highly desirable invention compositions, an antiperspirant active is present, either without or supplemented by the alternative deodorant active.

An essential constituent of compositions of the present invention is a non-ionic emulsifier or mixture of emulsifiers forming an emulsifier system. Such an emulsifier system conveniently has a mean HLB value in the region of from about 5 to about 12 and particularly from 6 to about 10. An especially desired mean HLB value is from 7m to 9. Such a mean HLB value can be provided by selecting an emulsifier having such an HLB value, or more preferably by employing a combination of at least two emulsifiers, a first (lower) HLB emulsifier having an HLB value in the range of from 2 to 6.5, such as in particular from 4 to 6 and a second (higher) HLB emulsifier having an HLB value in the range of from about 6.5 to 18 and especially from about 12 to about 18. When a combination of emulsifiers is employed, the average HLB value can be obtained by a weight average of the HLB values of the constituent emulsifiers.

An especially desirable range of emulsifiers comprise a hydrophilic moiety provided by a polyalkylene oxide (polyglycol), and a hydrophobic moiety provided by an aliphatic hydrocarbon, preferably containing at least 10 carbons and commonly linear. The hydrophobic and hydrophilic moieties can be linked via an ester or ether linkage, possibly via an intermediate polyol such as glycerol.

Preferably the hydrophobic aliphatic substituent contains at least 12 carbons, and is derivable from lauryl, palmityl, cetyl, stearyl, olearyl and behenyl alcohol, and especially cetyl, stearyl or a mixture of cetyl and stearyl alcohols or from the corresponding carboxylic acids. It is particularly convenient to employ an emulsifier comprising a polyalkylene oxide ether.

The polyalkylene oxide is often selected from polyethylene oxide and polypropylene oxide or a copolymer of ethylene oxide and comprises a polyethylene oxide. The number of alkylene oxide and especially of ethoxylate units within suitable emulsifiers is often selected within the range of from 2 to 100. Emulsifiers with a mean number of ethoxylate units in the region of 2 can provide a lower HLB value of below 6.5 and those having at least 4 such units a higher HLB value of above 6.5 and especially those containing at least 10 ethoxylate units. A preferred combination comprises a mixture of an ethoxylate containing 2 units and one containing from 10 to 40 units. Particularly conveniently, the combination of emulsifiers comprises steareth-2 and a selection from steareth-15 to steareth-30.

It is desirable to employ a mixture of ethoxylated alcohol emulsifiers in a weight ratio of emulsifier having a lower HLB value of <6.5 to emulsifier having a higher HLB value of >8 of from 1.5:1 to 6:1 and particularly from 2:1 to 5:1.

The total proportion of emulsifiers in the composition is usually at least 1.5% and particularly at least 2% by weight. Commonly the emulsifiers are not present at above 6%, often not more than 5% by weight and in many preferred embodiments up to 4% by weight. An especially desirable concentration range for the emulsifiers is from 2.5 to 4% by weight.

An other essential constituent of the present invention compositions is an oil. The oil is advantageously a plant oil and particularly is a triglyceride oil. Such oils are often obtainable by extraction from the plant's seeds. Suitable plant oils include sunflower seed oil, maize corn oil, evening primrose oil, coriander seed oil, safflower oil, olive oil, rape seed oil, castor oil and borage seed oil. It is particularly desirable to employ an oil which comprises mono or polyunsaturated long chain aliphatic carboxylate substituents, such as notably C18 carboxylates containing 1, 2 or 3 degrees of unsaturation, 2 or more or which may be conjugated. Other suitable oils which come into consideration include jojoba oil. The proportion of oil in the composition (excluding any contribution from water-insoluble constituents of fragrance oils which may be present) is often at least 1% and commonly a least 1.5% by weight. In many instances the proportion of oil is not more than 10% by weight and notably is not more than 5% by weight.

A further essential constituent of the composition comprises a particulate silica in the form of a fumed silica. It is particularly desirable to employ such a fumed (sometimes called pyrogenic) silica which has been hydrophobically treated. Such materials are commercially available under the name hydrophobic silica. Hydrophobic silicas are obtained by chemically bonding a hydrophbic substituent such as especially a siloxane group onto the surface of the silica, possibly following an intermediate treatment in which the surface of the silica has been rendered hydrophilic. Suitable reactants to generate a hydrophobic substituent include halosilanes and in particular chlorosilanes and methylated silazanes such as hexamethyldisilazane. It is particularly desirable to employ a silica that is capable of thickening an oil such as a plant oil.

Desirably, the silica, such as the fumed silica, and especially the hydrophobic silica has a BET specific surface area of at least 100 m²/g and particularly from 150 to 400 m²/g. The silica comprises very fine particles, fumed silica commonly having a diameter for individual particles of below 40 nm and in many instances at least 99% by weight of below 40 nm. In fumed silica as supplied, some aggregation can occur so that in many embodiments, the supplied silica has an average particle size (diameter) of less than or equal to 1000 nm, preferably less than or equal to 500 nm, i.e. the diameter of the silica particle of average weight. In at least some desirable embodiments, at least 99% by weight of the silica particles, as supplied, are in the range of 10 to 500 nm..

The weight proportion of silica in the formulation is often selected taking into account the desired viscosity of the eventual formulation, together with other attributes such as its effect on the speed of drying of the formulation, its perceived greasiness and/or its perceived stickiness. The weight concentration of silica in the composition is desirably at least 0.2%, often at least 0.3% and in many desirable embodiments is at least 0.5% by weight. Its concentration is commonly not greater than 2%, often not greater than 1.5% and in a number of very desirable formulations is not higher than 1.0%. A preferred weight range of silica concentrations is from 0.6 to 0.8%.

The water content of the composition is commonly selected in the range of from 65 to 93% by weight and often from 70 or 75 to 85% by weight.

The weight ratio of silica to water in the invention emulsions is commonly selected in the range of at least 1:400 up to 1:40, often at least 1:275 and in many instances preferably at least 1:200. It is often convenient to employ a weight ratio of up to 1:75.

In addition to the foregoing essential constituents it is preferable to include a fragrance, for example in a proportion of from 0.1 to 3% by weight, and particularly from 0.3 to 2% by weight.

In a number of highly desirable embodiments, the invention compositions comprise, by weight,:-
from 70 to 85% of water;
from 10 to 20% of an antiperspirant active, such as actives described hereinbefore;
from 2.5 to 4.0% of an ethoxylated ether emulsifier or mixture of emulsifiers, preferably having an HLB value of from 7 to 9;
from 1.5 to 4% by weight of a plant oil, such as an unsaturated fatty acid triglyceride;
from 0.5 to 1.0% of a hydrophobic fumed silica and from 3.3 to 2% of a fragrance.

By the selection of the proportions of the above identified constituents within the foregoing disclosed ranges of proportions, it is possible to obtain emulsions having a viscosity which fall within a preferred range of from 1000 to 7000 mPa.s and particularly within 2500 to 5500 mPa.s. Viscosities herein are measured in a Brookfield RVT viscometer equipped with a stirrer TA and Hellipath, rotating at 20 rpm at 25°C unless otherwise stated. Such emulsions demonstrate a particularly desirable combination of product attributes such as improved speed of drying, superior greasiness and avoidance of excessive stickiness on application.

According to a second aspect of the instant invention there is provided a process for making an emulsion in accordance with claim 27 herein. The steps of mixing and shearing can be carried out using the relevant conventional equipment.

Preferably, the emulsion is made by first preparing separate aqueous and oil mixtures which are brought together before shearing. The aqueous phase commonly contains the antiperspirant active. Where a mixed emulsifier system is employed, it is desirable to incorporate any emulsifier having a low HLB value, particularly of <6.5 into the oil phase and an emulsifier having a high HLB value, particularly of >6.5 into the aqueous phase. The temperature of the respective phases can be raised, where necessary, to accelerate dissolution of the emulsifier, for example to above 50°C.

It is highly desirable to incorporate the hydrophobic silica, with the aqueous phase.
It is preferable to incorporate any fragrance last of all and shortly before the entire mixture is sheared, especially when either or both phases have been heated so as to accelerate emulsifier dissolution.

The invention formulations are very suitable for dispensing via a roll-on dispenser, for example any dispenser such as described in EP12175165 or an invert dispenser such as described in USP6511243.

The instant invention provides an antiperspirant or deodorant composition which may be used in a dispenser comprising a reservoir containing an oil in water fluid emulsion composition as claimed
said reservoir having an outlet for the composition defining a housing for a roller
a roller retained by the housing and partially proud of the outlet
a means for releasably sealing the outlet and
a cap for the outlet having an exterior profile that permits the dispenser to stand stably in an invert orientation.

Upright herein indicates that when the dispenser is standing on a surface, the reservoir is below the housing for the roller, which in turn is below the cap, whereas Invert indicates the opposite.

The sealing means conveniently comprises a screw threaded connection between the interior of the cap and the exterior of the housing so as to urge the roller into fluid-tight contact with the housing. The roller most conveniently comprises a sphere and the housing most conveniently has a radiuses inner surface dimensioned so as to allow a small gap between the roller and the housing when the outlet is not sealed. When the roller is urged inwardly towards the housing, for example by suitably rotating the cap about the exterior of the housing, the screw threads engage, the cap urges the roller inwardly and the gap is sealed.

The invention formulation are particularly suited to being dispensed from a dispenser comprising one or more perturbators to regulate fluid flow and particularly gaseous flow within the housing for the ball and/or a means to control the depth of liquid adhering to the ball.

Having described the invention in general terms, specific embodiments will now be described in more detail by way of example only.

### Examples:

The Examples and comparison compositions were made by the following general method:-
An aqueous phase was prepared by mixing together in a vessel a 50% aqueous solution of an astringent antiperspirant active, water and any emulsifier having a high HLB value, (>6.5) and heating the mixture until the emulsifier dissolved, typically in the region of 55 to 65°C. Any silica was incorporated into this aqueous mixture. In a second vessel, an oil phase was prepared by mixing the selected oil with any emulsifier having a low HLB value (< 6.5) and heating the mixture until the emulsifier dissolved, conveniently also in the region of 55 to 65°C. The oil phase was then slowly introduced with continuous stirring into the first vessel. The resultant mixture was allowed to cool to below 40°C and any fragrance was added. The resultant mixture was then passed through a high shear mixer to form an emulsion and charged into roll-on dispensers as described in EP 1175165. Example 4 was made by a variation of the general method in which the hydrophobic silica was added in the oil phase.

The composition of products Ex 1 to 4 and Comp A are summarised in Table 1 below.

**Table 1**

| | Ex 1 | Ex 2 | Ex 3 | Ex 4 | CompA | ComB |
|---|---|---|---|---|---|---|
| Ingredient | % by weight | | | | | |
| Aluminum Chlorohydrate (50% w/w solution)*¹ | 30.0 | 30.0 | 30.0 | 30.0 | 30.0 | 30.0 |
| Steareth-2*² | 2.3 | 2.3 | 2.0 | 2.0 | 2.3 | 2.6 |
| Helianthus Annuus*³ | 2.0 | 2.0 | 4.0 | 4.0 | 2.0 | 4.0 |
| Steareth-20*⁴ | 0.9 | 0.9 | 0.5 | 0.5 | 0.9 | 0.6 |
| Hydrophobic Silica*⁵ | 0.7 | 0.3 | 0.7 | | | |
| Hydrophobic Silica*⁶ | | | | 0.7 | | |
| PVM/MA copolymer | | | | | 0.31 | 0.31 |
| solution*⁷ | | | | | | |
| fragrance | 0.8 | 0.8 | 0.8 | 0.8 | 0.8 | 0.8 |
| Water | balance to 100% | | | | | |

| | | | | | | |
|---|---|---|---|---|---|---|
| *¹ Chlorhydrol™ sol - Reheis *² Tego Alkanol S2™ - Degussa *³ high oleic - Henry Lamotte *⁴ Brij 78™ - Uniquema *⁵ HDK H30™ - Wacker Chemie *⁶ Aerosil R8200™ - Degussa *⁷ Gantrez S95™ sol - ISP | | | | | | |

All the Examples and comparison formulations had a viscosity in the range of 1500 to 5500 mPa.s at 25°C.

### Sensory test

The sensory properties of Comparison A and Examples 1 and 2 were then compared in a head to head test.

In the sensory test, 15 expert and experienced panellists assessed inter alia the greasiness of the formulations by stroking the roll-on across the underarm in a standardised way to deposit a dose of approximately 0.3g product, determined by weighing the dispenser before and after dose application. Products were blind coded, the order of test presentation fully randomised, and products were tested in duplicate, in order to increase data reliability. The panellists assessed greasiness on a scale of 1 to 100 on application and at minute intervals for a short while thereafter. The data was then analysed using a commercial statistical programme and the results on application and after 1 minute are summarised in Table 2 below, in which ^{SD} indicates that the improvement was statistically significant at the 95% confidence limit.

**Table 2**

| Formulation | Comp A | | Ex 1 | | Ex 2 | |
|---|---|---|---|---|---|---|
| | Mean | Std dev | Mean | Std dev | Mean | Std dev |
| Initial (t=0) | 29.33 | 8.38 | 27.00 | 6.45 | 25.13^{SD} | 5.64 |
| After 1 min | 28.27 | 7.56 | 23.80^{SD} | 7.49 | 23.87^{SD} | 6.63 |

Table 2 shows that the formulations incorporating the hydrophobised silica particles were less greasy than the comparison product Comp A which did not contain any silica particles, but otherwise contained the same proportions of the other active ingredients, water being considered not to be active. The improvement was at least directional on application and statistically significant after only 1 minute.

Similar sensory were carried out for Examples 3 and 4 compositions, and it was found that they again showed at least a directional improvement in greasiness, though tending to take longer to reach an improvement that was statistically significant than the formulations of Examples 1 or 2.

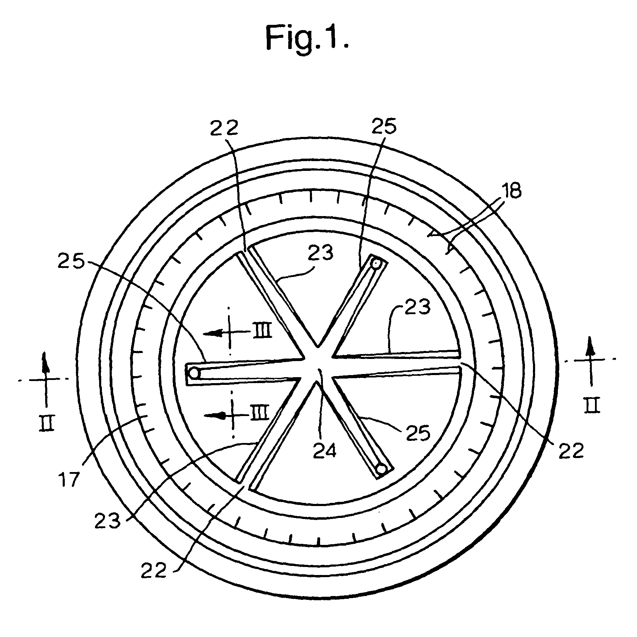

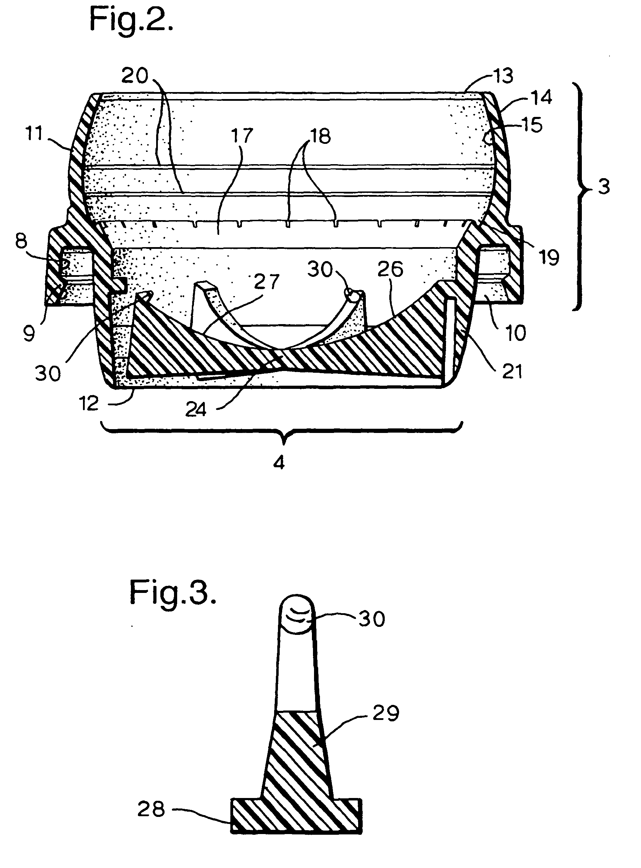

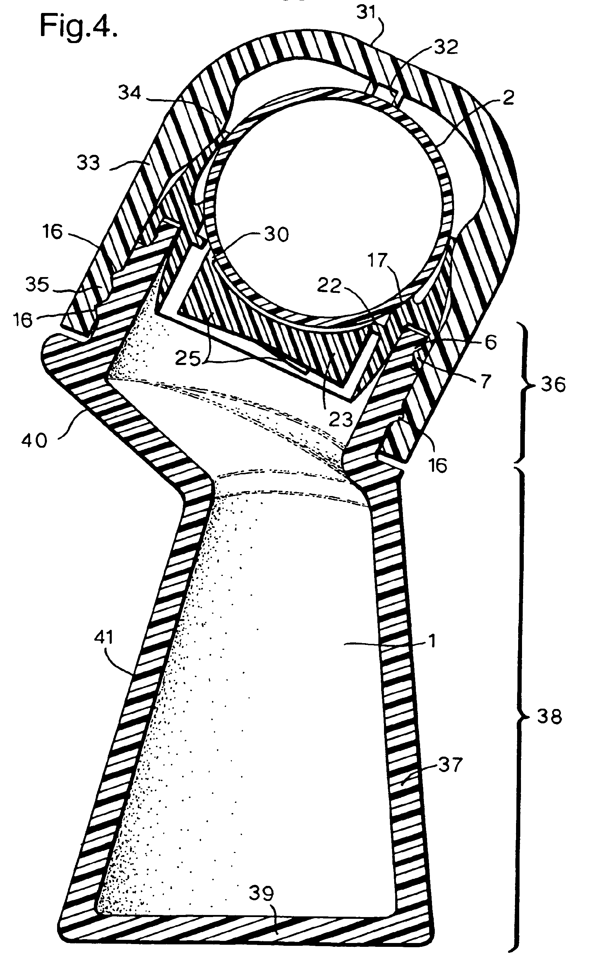

The actual and perceived speed of drying of the invention formulation of Example 1 dispensed from an applicator, as described herein with respect to Figures 1 to 4, was compared with a comparison formulation Comp B dispensed from a dispenser according to the example of WO 00/64302. The formulations were applied employing the same procedure as described previously herein for assessing their greasiness. Panellists determined the time of actual drying by touching the underarm and recording when the skin was dry to the touch of their fingers, and the perceived time of drying was recorded when panellists' skin sensation indicated to them that the underarm was dry, without touching it with their fingers. The recorded times were averaged and rounded to the nearest minute.

The comparison product Comp B achieved an actual speed of drying of 8 minutes and a perceived speed of drying of 9 minutes, whereas the invention product Ex 1 had an actual speed of drying of 6 minutes and a perceived speed of drying of 7 minutes. This shows that the invention product dried significantly more quickly than the comparison product, whether it was measured by touch or by skin sensation.

In the drawings for the dispenser of the invention composition employed in the drying test:-
Figure 1 is a plan view of a housing for a roll ball suitable for mounting on a cosmetic bottle, without the ball in place;
Figure 2 is a cross section view of the housing of Figure 1;
Figure 3 shows a transverse cross section through a free spoke shown in Figure 1;
Figure 4 is a cross section view of a dispenser showing the housing of Figures 1 and 2 with ball in place, mounted on a bottle and having a cap screwed tight.

As illustrated in the Figures, the roll-on dispenser comprised a bottle (1), a spherical ball (2), a housing (3) for the ball (2) integrally moulded with a spider (4) and a cap (5), each of which are moulded from a thermoplastic polymer.

The bottle (1) at its open end has an exterior annular lip (6) and annular groove (7) which snap fit with co-operating annular recess (8) and bead (9) moulding on the inward-face of an annular channel (10) formed by a bifurcated side-wall of the housing (3) dimensioned for a fluid tight fit with the bottle.

The housing (3) comprises an annular side-wall (11) of circular lateral cross section extending between an inward end (12) and an outward end (13). The side wall (11) comprises an upper wall of tapering cross section (14) adjacent to the outward end (13) which has a concave interior face (15) having two lateral shallow beads (20) and screw threads (16) on an exterior face. Shallow annular beads (20) act as baffles which perturbs air as it flows into the bottle across the interior face of the housing during use and thereby smooth the application of liquid. The housing (3) has a thickened middle wall section having an inward facing annular sealing ring (17) into the outward (upper) edge of which are cut a multiplicity of short notches (18) equidistantly spaced around the ring extending down about 30% of the axial height of the sealing ring (17), which disrupt the flow of air across the interior face of the housing. The notches (18) are of about the same depth as and in fluid communication with a lateral annular v-shaped groove (19) is defined by an inward face of the upper wall (11) and an outward face of the ring (17), which also provides a small intermediate reservoir for liquid when the dispenser is in an upright orientation. The housing side-wall is bifurcated, providing an inner annular skirt (21) extends into the bottle (1) from the middle section of the housing (3) on which a spider (4) is mounted at three equidistant points (22) around the skirt (21), which spider (4) extends across the inward end (12) of the housing.

The spider (4) comprises three fixed arms (23) extending from the housing skirt (21) to a hub (24) from which radiates three free spokes (25) that each are equidistant from the adjacent fixed spokes (23) and extend about 90% of the distance from the hub to the interior face of the skirt. Each respective spoke (23), (25) has respective concave faces (26) and (27) that face the ball (2) in the housing which has a similar radius of curvature to that of the ball, and is of T-shaped cross section having a strengthening base flanges (28) from which a tapering wall (29) extends upwardly. Each free spoke has at it free end a pimple (30) proud of the concave face (27) which spaces that face (27) from the ball (2) and the pimples (30), being symmetrically arranged, centre the ball. The spokes (23, 25) act as wiper blades regulating the depth of film adhering to the surface of the ball (2) as the ball rotates.

The bottle (1) comprises a head section (36) of circular cross section having an axis inclined at an angle of about 22° to a front wall (37) of a body (38) of oval transverse cross section, which front wall is almost perpendicular to flat base (39) that permits the dispenser to stand upright. To its rearward side, head (35) has a more sharply inclined base wall (40) that extends approximately to beneath the axis of the head (36) and intersects with a sloping rear wall of the body (38) which is approximately aligned with the head axis. The head base wall (40) forms with body rear side-wall (41) a shelf to support the index finger of the hand that grasps the dispenser in a handshake, rear side-wall (41) being pressed into the palm of the hand.

The cap (5) has a top-wall (31) having a flat outer surface enabling the dispenser to be stood in an invert orientation and a centrally located annular dependent wall (32) which can contact the ball (2) to urge it against the sealing ring (17) and a side-wall (33) having on its interior face an inward-facing annular shoulder (34) which can contact the upper wall (14 of the housing (3) and urge it against the ball (2) and on its exterior face a screw thread (35) for co-operation with a corresponding thread (16) on the bottle head (36). When the cap (5) is being fitted to the bottle (1) over housing (3), the cap rotation around the housing is converted by the co-operating screw threads (16, 35) into relative axial movement, so that the ball (2) is urged by annular wall (32) towards the bottle (1) and likewise shoulder (34) acting against upper wall (15) of the housing (3) in turn acts on ball (2). In turn, the ball (2) being in contact with the pimples (30) on the free spokes (25) flexes the spider (4) and in particular the free spokes (25) towards the interior of the bottle (1). When the cap is removed, the spider (4) and in particular the free spokes (25) return to their rest positions due to their resilient character. In that rest position, the concave face (26, 27) of the spokes (23) and (25) create with the ball outer surface an annular gap (36) of reasonably constant depth defined by the height of the pimples (30). When the dispenser is in its upright orientation and as the ball(2) rotates) it encounters the leading edges of the spokes (23) and (25) and excess liquid is wiped away leaving a film of desired thickness on the ball, the remainder falling back into the bottle (1).

## Claims

1. An antiperspirant or deodorant composition in the form of an oil-in-water emulsion comprising:-
a continuous aqueous phase in which is dissolved or dispersed an antiperspirant or deodorant active;
a dispersed oil phase;
a nonionic emulsifier or mixture of emulsifiers; and
as an essential constituent a dispersed particulate fumed silica, in an amount of not greater than 2% by weight of the composition, wherein the fumed silica comprises hydrophobic silica.

2. A composition according to claim 1 in which the emulsifier or mixture comprises an alkoxylated aliphatic alcohol.

3. A composition according to claim 2 in which the emulsifier or mixture comprises a ceteth, steareth or ceteareth emulsifier.

4. A composition according to any preceding claim in which the emulsifier or mixture has an average degree of ethoxylation of from 5 to 12.

5. A composition according to claim 4 in which the emulsifier or mixture has an average degree of ethoxylation of from 7 to 9.

6. A composition according to any of claims 1 to 3 in which the emulsifier or mixture of emulsifiers has a mean HLB value of from 5 to 12.

7. A composition according to claim 6 in which the emulsifier or mixture of emulsifiers has a mean HLB value of from 7 to 9.

8. A composition according to any of claims 3 to 7 in which the mixture of emulsifiers comprises a mixture of a tower HLB emulsifier having a degree of ethoxylation of from 2 or 3 and a higher HLB emulsifier having a degree of ethoxylation of from 10 to 40.

9. A composition according to claim 8 in which the first and second emulsifiers are present in a weight ratio of from 2:1 to 5:1.

10. A composition according to any preceding claim in which the proportion of the emulsifier is from 2 to 4% by weight.

11. A composition according to any preceding claim in which the oil phase comprises a plant oil.

12. A composition according to claim 10 in which the plant oil is a triglyceride oil.

13. A composition according to any preceding claim containing from 1 to 5% by weigh of the oil.

14. A composition according to claim 12 containing from 1.5 to 4% by weight of the oil.

15. A composition according to any preceding claim which contains at least 0.1% by weight of the silica.

16. A composition according to claim 15 which contains at least 0.2% by weight of the silica.

17. A composition according to claim 16 which contains from 0.3% by weight of the silica.

18. A composition according to claim 17 which contains from 0.3 to 1.5% by weight of the silica.

19. A composition according to claim 18 containing from 0.5 to 1.0% by weight of the silica.

20. A composition according to any preceding claim in which the silica has an average particle size of up to 500 nm.

21. A composition according to any preceding claim in which the silica is present in a weight ratio to water of from 1:275 to 1:75.

22. A composition according to any preceding claim which contains from 0.3 to 1.5% by weight of a fragrance.

23. A composition according to any preceding claim in which the total weight proportion of emulsifiers and oils is not greater than 7.5%.

24. A composition according to claim 23 in which said weight proportion is from 5.5 to 7.0%.

25. A composition according to claim 23 or 24 which contains at least one higher HLB emulsifier of >8 in a weight proportion of from 0.5 to 1.0%.

26. A composition according to any preceding claim having a viscosity of from 1800 to 5500 mPA.s at 25°C.

27. A process for making an antiperspirant or deodorant oil-in-water emulsion comprising as ingredients water, an antiperspirant or deodorant active, a nonionic oil-in-water emulsifier or mixture of emulsifiers, an oil and a particulate hydrophic fumed silica, which process comprises the step of mixing together the ingredients and shearing the mixture to disperse the oil as droplets within the water, **characterised in that** the resultant composition contains not greater than 2% by weight of a particulate hydrophobic fumed silica and is in accordance with any one preceding claim.

28. A process according to claim 27 in which the mixture of ingredients is obtained by preparing an aqueous phase and an oil phase separately and mixing the two phases prior to shearing the mixture of phases.

29. A process according to claim 28 employing the mixture of emulsifiers in which one having an HLB value of <6.5 is incorporated with the oil phase and a second having an HLB of >6.5 is incorporated with the aqueous phase.

30. A process according to claim 28 or 29 in which the silica is incorporated with the aqueous phase.

## Patentansprüche

1. Antiperspirant- oder Deodorant-Zusammensetzung in der Form einer Öl-in-Wasser-Emulsion, umfassend:
eine kontinuierliche wässrige Phase, in der ein Antiperspirant- oder Deodorant-Wirkstoff gelöst oder dispergiert ist;
eine dispergierte Ölphase;
ein nicht-ionischer Emulgator oder ein Gemisch von Emulgatoren und
als essentieller Bestandteil ein dispergiertes partikelförmiges hoch-disperses Siliciumdioxid in einer Menge von nicht größer als 2 Gewichts-% der Zusammensetzung, wobei das hoch disperse Siliciumdioxid hydrophobes Siliciumdioxid umfasst.

2. Zusammensetzung nach Anspruch 1, wobei der Emulgator oder das Gemisch einen alkoxylierten aliphatischen Alkohol umfasst.

3. Zusammensetzung nach Anspruch 2, wobei der Emulgator oder das Gemisch einen Ceteth-, Steareth- oder Ceteareth-Emulgator umfasst.

4. Zusammensetzung nach einem vorangehenden Anspruch, wobei der Emulgator oder das Gemisch einen durchschnittlichen Ethoxylierungsgrad von 5 bis 12 hat.

5. Zusammensetzung nach Anspruch 4, wobei der Emulgator oder das Gemisch einen durchschnittlichen Ethoxylierungsgrad von 7 bis 9 hat.

6. Zusammensetzung nach einem der Ansprüche 1 bis 3, wobei der Emulgator oder das Gemisch von Emulgatoren einen mittleren HLB-Wert von 5 bis 12 hat.

7. Zusammensetzung nach Anspruch 6, wobei der Emulgator oder das Gemisch von Emulgatoren einen mittleren HLB-Wert von 7 bis 9 hat.

8. Zusammensetzung nach einem der Ansprüche 3 bis 7, wobei das Gemisch von Emulgatoren ein Gemisch aus einem Emulgator mit niedrigerem HLB-Wert, der einen Ethoxylierungsgrad von 2 oder 3 hat, und einem Emulgator mit höherem HLB-Wert, der einen Ethoxylierungsgrad von 10 bis 40 hat, umfasst.

9. Zusammensetzung nach Anspruch 8, wobei der erste und zweite Emulgator in einem Gewichtsverhältnis von 2:1 bis 5:1 vorliegen.

10. Zusammensetzung nach einem vorangehenden Anspruch, wobei der Verhältnisanteil des Emulgators 2 bis 4 Gewichts-% ist.

11. Zusammensetzung nach einem vorangehenden Anspruch, wobei die Ölphase ein Pflanzenöl umfasst.

12. Zusammensetzung nach Anspruch 10, wobei das Pflanzenöl ein Triglyceridöl ist.

13. Zusammensetzung nach einem vorangehenden Anspruch, die 1 bis 5 Gewichts-% des Öls enthält.

14. Zusammensetzung nach Anspruch 12, die 1,5 bis 4 Gewichts-% des Öls enthält.

15. Zusammensetzung nach einem vorangehenden Anspruch, die wenigstens 0,1 Gewichts-% des Siliciumdioxids enthält.

16. Zusammensetzung nach Anspruch 15, die wenigstens 0,2 Gewichts-% des Siliciumdioxids enthält.

17. Zusammensetzung nach Anspruch 16, die 0,3 Gewichts-% des Siliciumdioxids enthält.

18. Zusammensetzung nach Anspruch 17, die 0,3 bis 1,5 Gewichts-% des Siliciumdioxids enthält.

19. Zusammensetzung nach Anspruch 18, die 0,5 bis 1,0 Gewichts-% des Siliciumdioxids enthält.

20. Zusammensetzung nach einem vorangehenden Anspruch, wobei das Siliciumdioxid eine durchschnittliche Partikelgröße von bis zu 500 nm hat.

21. Zusammensetzung nach einem vorangehenden Anspruch, wobei das Siliciumdioxid in einem Gewichtsverhältnis zu Wasser von 1:275 bis 1:75 vorliegt.

22. Zusammensetzung nach einem vorangehenden Anspruch, die 0,3 bis 1,5 Gewichts-% eines Duftmittels enthält.

23. Zusammensetzung nach einem vorangehenden Anspruch, wobei der Gesamtgewichtsverhältnisanteil von Emulgatoren und Ölen nicht größer als 7,5 % ist.

24. Zusammensetzung nach Anspruch 23, wobei der Gewichtsverhältnisanteil von 5,5 bis 7,0 % ist.

25. Zusammensetzung nach Anspruch 23 oder 24, die wenigstens einen Emulgator mit höherem HLB-Wert von > 8 in einem Gewichtsverhältnisanteil von 0,5 bis 1,0 % enthält.

26. Zusammensetzung nach einem vorangehenden Anspruch, die eine Viskosität von 1800 bis 5500 mPA.s bei 25 °C hat.

27. Verfahren zur Herstellung einer Antiperspirant- oder Deodorant-Öl-in-Wasser-Emulsion, umfassend als Ingredienzien Wasser, einen Antiperspirant- oder Deodorant-Wirkstoff, einen nicht-ionischen Öl-in-Wasser-Emulgator oder ein Gemisch von Emulgatoren, ein Öl und ein partikuläres hydrophobes hoch-disperses Siliciumdioxid, wobei das Verfahren den Schritt des Zusammenmischens der Ingredienzien und des Scherens des Gemisches, um das Öl als Tröpfchen im Wasser zu dispergieren, umfasst, **dadurch gekennzeichnet, dass** die resultierende Zusammensetzung nicht mehr als 2 Gewichts-% eines partikulären hydrophoben hoch-dispersen Siliciumdioxids enthält und einem vorangehenden Anspruch entspricht.

28. Verfahren nach Anspruch 27, wobei das Gemisch von Ingredienzien durch Herstellen einer wässrigen Phase und einer Ölphase getrennt und Vermischen der zwei Phasen vor Scherung des Gemisches von Phasen erhalten wird.

29. Verfahren nach Anspruch 28, das das Gemisch von Emulgatoren verwendet, indem einer, der einen HLB-Wert von < 6,5 hat, in die Ölphase eingearbeitet wird und ein zweiter, der einen HLB-Wert von > 6,5 hat, in die wässrige Phase eingearbeitet wird.

30. Verfahren nach Anspruch 28 oder 29, wobei das Siliciumdioxid in die wässrige Phase eingearbeitet wird.

## Revendications

1. Composition anti-transpirante ou déodorante sous la forme d'une émulsion huile dans l'eau comprenant :
■ une phase aqueuse continue dans laquelle un agent anti-transpirant ou déodorant actif est dissous ou dispersé ;
■ une phase huileuse dispersée ;
■ un émulsifiant ou un mélange d'émulsifiants non ionique(s) ; et
■ à titre de constituant essentiel, de la silice fumée particulaire dispersée, en une quantité n'excédant pas 2 % en poids de la composition, dans laquelle la silice fumée comprend une silice hydrophobe.

2. Composition selon la revendication 1, dans laquelle l'émulsifiant ou le mélange comprend un alcool aliphatique alcoxylé.

3. Composition selon la revendication 2, dans laquelle l'émulsifiant ou le mélange comprend un émulsifiant du type Ceteth, Steareth ou Ceteareth.

4. Composition selon l'une quelconque des revendications précédentes, dans laquelle l'émulsifiant ou le mélange a un degré moyen d'éthoxylation de 5 à 12.

5. Composition selon la revendication 4, dans laquelle l'émulsifiant ou le mélange a un degré moyen d'éthoxylation de 7 à 9.

6. Composition selon l'une quelconque des revendications 1 à 3, dans laquelle l'émulsifiant ou le mélange d'émulsifiants a un indice HLB moyen variant de 5 à 12.

7. Composition selon la revendication 6, dans laquelle l'émulsifiant ou le mélange d'émulsifiants a un indice HLB moyen variant de 7 à 9.

8. Composition selon l'une quelconque des revendications 3 à 7, dans laquelle le mélange d'émulsifiants comprend un mélange d'un émulsifiant à bas indice HLB ayant un degré d'éthoxylation variant de 2 ou 3 et d'un émulsifiant à indice HLB élevé ayant un degré d'éthoxylation variant de 10 à 40.

9. Composition selon la revendication 8, dans laquelle les premiers et seconds émulsifiants sont présents dans un rapport en poids variant de 2/1 à 5/1.

10. Composition selon l'une quelconque des revendications précédentes, dans laquelle la proportion de l'émulsifiant varie de 2 à 4 % en poids.

11. Composition selon l'une quelconque des revendications précédentes, dans laquelle la phase huileuse comprend une huile végétale.

12. Composition selon la revendication 10, dans laquelle l'huile végétale est une huile tri-glycéridique.

13. Composition selon l'une quelconque des revendications précédentes contenant de 1 à 5 % en poids d'huile.

14. Composition selon la revendication 12 contenant de 1,5 à 4 % en poids d'huile.

15. Composition selon l'une quelconque des revendications précédentes qui contient au moins 0,1 % en poids de silice.

16. Composition selon la revendication 15 qui contient au moins 0,2 % en poids de silice.

17. Composition selon la revendication 16 qui contient à partir de 0,3 % en poids de silice.

18. Composition selon la revendication 17 qui contient de 0,3 à 1,5 % en poids de silice.

19. Composition selon la revendication 18 qui contient de 0,5 à 1,0 % en poids de silice.

20. Composition selon l'une quelconque des revendications précédentes, dans laquelle la silice a une taille de particules moyenne pouvant aller jusqu'à 500 nm.

21. Composition selon l'une quelconque des revendications précédentes, dans laquelle la silice est présente dans un rapport en poids à l'eau variant de 1/275 à 1/75.

22. Composition selon l'une quelconque des revendications précédentes qui contient de 0,3 à 1,5 % en poids de fragrance.

23. Composition selon l'une quelconque des revendications précédentes, dans laquelle la proportion en poids totale des émulsifiants n'est pas supérieure à 7,5 %.

24. Composition selon la revendication 23, dans laquelle ladite proportion en poids varie de 5,5 à 7,0 %.

25. Composition selon les revendications 23 ou 24 qui contient au moins un émulsifiant à indice HLB élevé, > 8, dans une proportion en poids variant de 0,5 à 1,0 %.

26. Composition selon l'une quelconque des revendications précédentes ayant une viscosité variant de 1800 à 5500 mPA.s à 25 °C.

27. Procédé de préparation d'une émulsion anti-transpirante ou déodorante du type huile dans l'eau comprenant à titre d'ingrédients, de l'eau, un agent anti-transpirant ou déodorant actif, un émulsifiant ou un mélange d'émulsifiants non ionique(s) du type émulsion huile dans l'eau, une huile et une silice fumée hydrophobe particulaire, ledit procédé comprenant l'étape consistant à mélanger les ingrédients ensemble et à soumettre le mélange à cisaillement pour disperser l'huile sous forme de gouttelettes dans l'eau, **caractérisé en ce que** la composition obtenue ne contient pas plus de 2 % en poids de silice fumée hydrophobe particulaire et est telle que définie dans l'une quelconque des revendications précédentes.

28. Procédé selon la revendication 27, dans laquelle le mélange d'ingrédients est obtenu en préparant une phase aqueuse et une phase huileuse séparément et en mélangeant les deux phases avant de soumettre le mélange de phases à cisaillement.

29. Procédé selon la revendication 28 utilisant le mélange d'émulsifiants dans lequel un émulsifiant ayant un indice HLB < 6,5 est incorporé avec la phase huileuse et un second émulsifiant ayant un indice HLB > 6,5 est incorporé avec la phase aqueuse.

30. Procédé selon les revendications 28 ou 29, dans lequel la silice est incorporée avec la phase aqueuse.
